# EUROPEAN PATENT APPLICATION

(11) **EP 3 744 250 A1**
(43) Date of publication of application: **02.12.2020**
(21) Application number: 20175268.0
(22) Date of filing: 18.05.2020
(51) Int. Cl.: A61B 5/1455, A61B 5/024

(54) **PHOTOPLETHYSMOGRAPHY DEVICE WITH A COMPENSATION CURRENT SOURCE**

(30) Priority: 30.05.2019 US 201962854479 P; 13.06.2019 US 201962860851 P
(71) Applicant: Welch Allyn, Inc., Skaneateles Falls, NY 13153 (US)
(72) Inventor: BREMER, Edward C., Skaneateles Falls, NY New York 13153 (US)
(74) Representative: Findlay, Alice Rosemary

(57) **Abstract**

A photoplesythmography (PPG) device includes an array of light emitting diodes (LED's) arranged to illuminate a tissue sample, and an array of photodetectors (PD's) adapted to detect light returned from the tissue sample and to output a PD output signal which depends at least in part on a bias current. The PPG device also includes a compensation current source and a controller. The controller is adapted to operate the compensation current source so that the current source outputs the bias current to the photodetector array.

## Description

The subject matter described herein relates to photoplethysmography (PPG) devices and in particular to a PPG device having a light emitter such as a light emitting diode (LED), a photodetector (PD) for receiving a return light signal, and an analogue to digital converter for converting an analogue photodetector output to a digital signal, the PPG device includes a compensation current source which provides a bias current to the PD so that the PD can receive an amplified return light signal and output a counterpart amplified output signal which will not saturate the ADC.

Photoplethysmography (PPG) is a simple, low cost, noninvasive technique used for a variety of purposes in the medical arts. The uses of PPG devices include monitoring blood oxygen saturation, blood pressure, heart rate, respiration, and cardiac output.

In practice a PPG device illuminates a tissue sample with light of different wavelengths (often red and infrared). A photodetector (PD) detects the amount of red and infrared light transmitted through or reflected by the tissue. An analogue to digital converter (ADC) converts the analogue outputs of the photodetector to digital "count" signals. A processor executes machine executable instructions to estimate a physiological parameter of interest based on the detected amount of red and infrared light, i.e. based on the count output of the ADC.

One challenge associated with PPG devices is that the light signal received at the photodetector contains both a static component and a fluctuating component (often referred to as DC and AC components). Typically the AC component contains the information of interest. However the AC component is quite small in comparison the the DC component (on the order of 1% of the DC component). As a result the information bearing AC component of the signal may be difficult to detect and its information content may be difficult to extract.

One way to attempt to overcome the above problem is to increase the drive current to the LED thereby amplifying the light signal received at the PD. Doing so amplifies the signal from the PD to the ADC and consequently increases the count output of the ADC for any given ADC input (PD output), resulting in improved resolution of the ADC count output. However it may also saturate the ADC. That is, the count output corresponding to the amplified input to the ADC may be greater than the maximum count output capability of the ADC.

Therefore, what is needed is a PPG device whose PD can receive an amplified light signal and output a counterpart amplified PD output signal which will not saturate the ADC.

An apparatus, system, or method may comprise one or more of the following features, alone or in any combination.

According to a first aspect of the present disclosure, a photoplesythmography (PPG) device may include an array of light emitting diodes (LED's) that may be arranged to illuminate a tissue sample. The PPG device may also include an array of photodetectors (PD's) that may be adapted to detect light that may be returned from the tissue sample and to output a PD output signal which may depend at least in part on a bias current. The PPG device may further have a compensation current source and a controller that may be adapted to operate the compensation current source so that the compensation current source may output the bias current to the array of photodetectors.

In some embodiments of the first aspect, the PPG device may further include an analogue to digital converter (ADC) which may process the PD output signal. Optionally, the controller may control the compensation current source so that a magnitude of the bias current may be a function of the ADC output signal. Further optionally, the controller may control the compensation current source such that the PD output signal may be within a range of operation of the ADC.

If desired, the compensation current source of the PPG device of the first aspect may include a digital to analogue converter (DAC). Alternatively or additionally, the compensation current source may include a digital potentiometer whose setting may be controlled by the controller to regulate the bias current. Further alternatively or additionally, the compensation current source may include a transistor. In such embodiments, the controller may control the transistor by way of a potentiometer that may be in electrical communication with the transistor.

In some embodiments of the PPG device of the first aspect, the controller may be adapted to: assess whether or not an ADC component of the PPG device may be saturated by the PD output signal and if the assessment determines that the ADC is saturated, the controller may command the compensation current source to increment its output. The controller of the first aspect may further test whether or not the ADC may have become unsaturated in response to the command to the compensation current source to increment its output. If the test reveals that the bias current resulting from the incremental adjustment may have unsaturated the ADC, the controller may decline to command a further increment to the output of the compensation current source. On the other hand, if the test reveals that the bias current resulting from the incremental adjustment may have not unsaturated the ADC, the controller may command a further incremental adjustment to the compensation current.

According to a second aspect of the present disclosure, a method of overcoming saturation of an analogue to digital converter (ADC) of a photoplethsymography (PPG) device may include assessing whether or not the ADC may be saturated by a photodetector output. If the assessment determines that the ADC may be saturated, the method of the second aspect may further include adjusting a compensation current by an incremental amount. The method of the second aspect may also include testing whether or not the ADC may have become unsaturated in response to the adjusting step. If the testing step reveals that the compensation current resulting from the incremental adjustment has unsaturated the ADC, the method of the second aspect may further include holding the compensation current at the amplitude resulting from the incremental adjustment. On the other hand, if the testing step reveals that the compensation current resulting from the incremental adjustment may have not unsaturated the ADC, the method of second aspect may further include making a further incremental adjustment to the compensation current.

In some embodiments, the method of the second aspect may further include illuminating a tissue sample with an array of light emitting diodes (LED's), detecting light returned from the tissue sample by an array of photodetectors (PD's), and outputting the photodetector output as a photodetector (PD) output signal. If desired, the method of the second aspect may include outputting the compensation current from a compensation current source and using a controller to operate the compensation current source so that the compensation current source may output the compensation current to the array of photodetectors.

Optionally, the method of the second aspect may further include controlling the compensation current source with the controller so that a magnitude of the compensation current may be a function of an ADC output signal from the ADC. Alternatively or additionally, the method of the second aspect may also include controlling the compensation current source with the controller such that the PD output signal may be within a range of operation of the ADC.

If desired, the compensation current source of the second aspect may include a digital to analogue converter (DAC). Alternatively or additionally, the compensation current source of the second aspect may include a digital potentiometer whose setting may be controlled by the controller to regulate the compensation current. Further alternatively or additionally, the compensation current source may include a transistor. In such embodiments, the controller of the second aspect may control the transistor by way of a potentiometer in electrical communication with the transistor.

The invention will now be further described by way of example with reference to the accompanying drawings, in which:
Fig. 1 is a side elevation view of a PPG device embodied as a reflectance blood oxygen saturation sensor and shown applied to a person's finger;
Fig. 2 is a view similar to Fif. 1 in which the PPG device is a transmittance blood oxygen saturation sensor;
Fig. 3 is a diagram of a PPG device which includes a compensation current source;
Figs. 4 and 5 are diagrams comparing the operational behavior of a PPG device having a compensation current source (Fig. 5) with one that does not have a compensation current source (Fig. 4);
Fig. 6 is a graph showing bias current as a function of photodetector output;
Fig. 7 is a diagram showing components and interconnections of Fig. 3 in greater detail; and
Fig. 8 is a block diagram illustrating a method of overcoming saturation of an analogue to digital converter of a PPG device.

In this specification and drawings, features similar to or the same as features already described may be identified by reference characters or numerals which are the same as or similar to those previously used. Similar elements may be identified by a common reference character or numeral, with suffixes being used to refer to specific occurrences of the element.

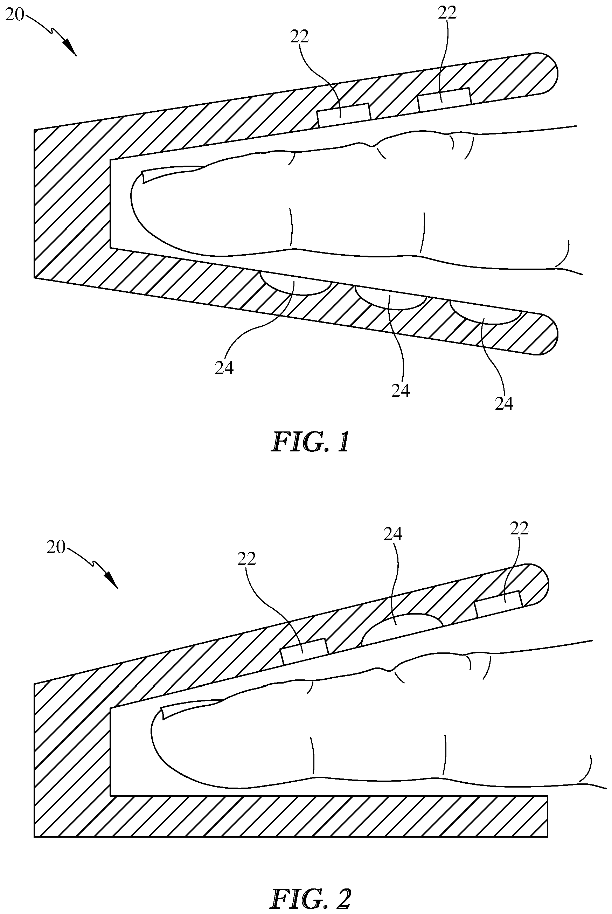

Fig. 1 shows a photoplesythmography (PPG) device 20 embodied as a blood oxygen saturation sensor that clamps onto a person's finger. The PPG device includes an array of light emitting diodes (LED's) 24 arranged to illuminate a tissue sample. Typically the LED array comprises at least one LED capable of emitting visible red light (e.g. at about 660 nm) and infrared light (e.g. at about 940 nm). Multiple LED's may be used such as the two LED's illustrated, one of which emits at 660 nm and the other of which emits at 940 nm. More than two LED's may be used to provide for multiple sources of light at 660 and/or at 940 nm. As is evident from the foregoing, "light" as used in this specification includes electromagnetic radiation outside the visible wavelengths of the electromagnetic spectrum.

The PPG device also includes an array of photodetectors (PD's) 24 adapted to detect light returned from the tissue sample as a result of the tissue having been illuminated with the emitted light from the LED's. The array may include a single PD or may include two or more PD's. In operation, the PPG device makes use of the fact that different tissues differ in the amount of red and infrared light they absorb. Therefore, the returned light has information content about the tissue. For example if the PPG device is designed as an oxygen saturation (SpO2) sensor, it makes use of the fact that oxygenated hemoglobin and deoxygenated hemoglobin have different red and infrared light absorption characteristics. These differences cause corresponding differences in the amount of red and infrared light returned to the PD. The differences in the return light are used by a processor to estimate the patient's blood oxygen saturation.

The LED array and the PD array may be referred to herein as simply "LED" and "PD."

The device of Fig. 1 is illustrated as having been applied to a patient's finger, as is frequently the case if the PPG device is designed as an oxygen saturation sensor. Therefore, the illuminated tissue sample in the example of Fig. 1 is tissue in the patient's finger (e.g. flesh, bone, venous blood, pulsatile arterial blood, and nonpulsatile arterial blood). PPG devices can also be used at other locations on the patient's body and for reasons other than SpO2 measurement.

In the device of Fig. 1 the LED and the PD are on opposite sides of the patient's finger. The device is therefore referred to as a transmittance type PPG device because the light returned to and detected by the PD is that portion of the emitted light that has penetrated through the tissue and arrived at the PD rather than having been absorbed or reflected by the tissue between the LED and the PD.

Fig. 2 shows another PPG device. The device of Fig. 2 is similar to that of Fig. 1 except that the LED and the PD are on the same side of the patient's finger. The device is therefore referred to as a reflectance type PPG device because the light returned to and detected by the PD is that portion of the emitted light that has reflected from the tissue and arrived back at the PD.

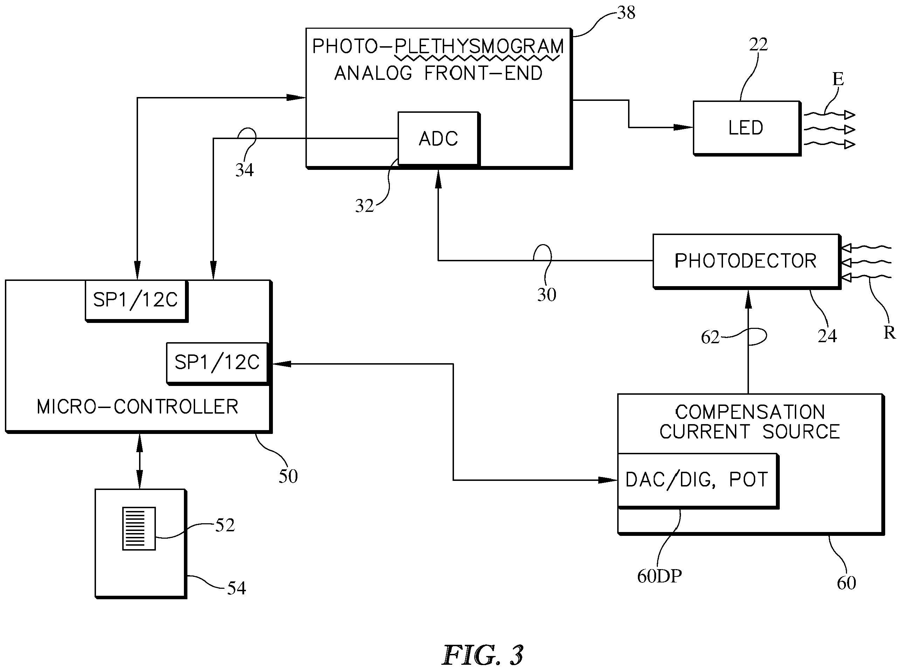

Fig. 3 is a diagram of a PPG device. The device includes an LED 22 which emits light E into tissue and a PD 24 which detects light R returned (reflected from or transmitted through) the tissue as already described. Whether the PPG device is a reflectance or transmittance device, the PD responds to the detected light by producing a PD output signal 30. The detected light R contains information about the illuminated tissue, and therefore so does PD output signal 30. An analogue to digital converter (ADC) 32 receives the PD output signal (electrical current) and processes it to convert it to a digital signal, specifically an ADC "count" illustrated as ADC output 34. In Fig. 3 the ADC is shown as a component of an analogue front end device 38.

A controller or processor 50 uses processor executable instructions 52 stored in a memory 54 to estimate a physiological parameter of interest, for example SpO2. In other words the instructions, when executed by the processor, estimate the parameter of interest. The controller includes a serial/peripheral interface (SPI) or an I2C bus for connection to front end device 38.

The PPG device also includes a compensation current source 60. In one embodiment the compensation current source is a digital potentiometer 60DP (referred to informally as a digipot). Controller 50 is adapted to operate the compensation current source 60 so that the current source 60 outputs a bias current or compensation current 62 to the photodetector array. In other words the setting of the digital potentiometer 60DP is controlled by the controller 50 to regulate the bias current 62. The bias current 62 is a negative current that at least partly oppositely compensates the DC component of the PD electrical current output 30. By doing so the bias current overcomes the potential problem of saturating the ADC 32, but nevertheless enables the ADC 32 to provide an output count whose resolution is better than would be the case if the bias current 62 were not applied.

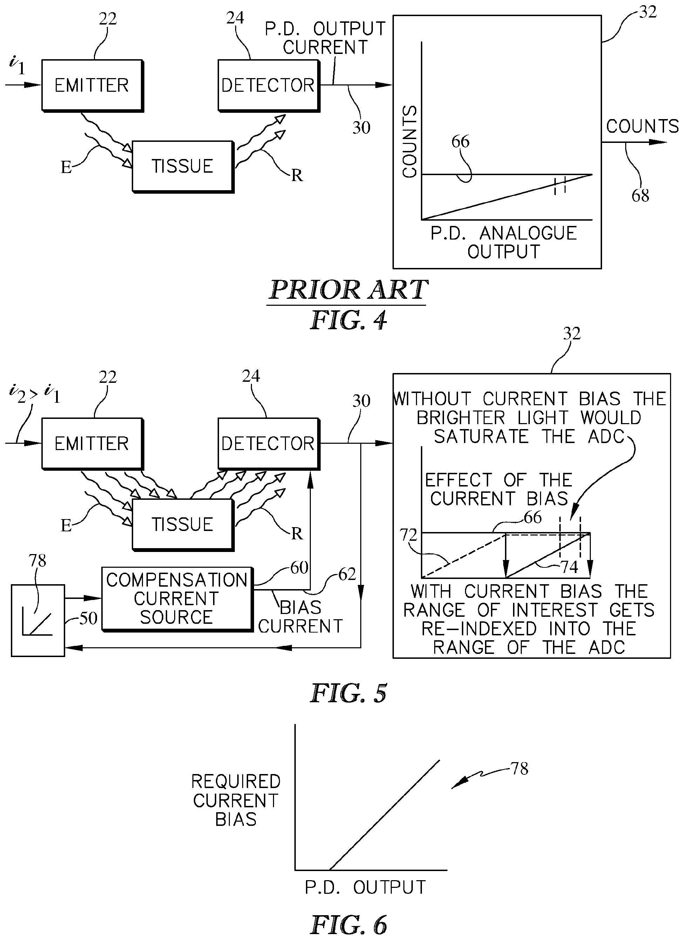

The foregoing is illustrated in Fig. 5 in comparison to Fig. 4. Beginning with Fig. 4, electrical current i₁ drives LED 22. The light emission E from the LED 22 illuminates the patient's tissue. Return light R is detected by photodetector 24. ADC 32, depicted as a graph of count vs. PD output, receives a PD output current corresponding to the detected return light R. Horizontal line 66 of the graph represents the maximum output capacity of the ADC 32. The ADC 32 converts the analogue current to a digital count 68. The conversion relationship is illustrated as linear, but need not be. As seen in the graph representing the ADC 32, the information bearing AC portion of the PD output signal (bounded by two vertical hash marks) is small in comparison to the DC component of the signal. Indeed, the AC component is exaggerated in the drawing to render it more easily discernible to the reader. The AC component is therefore difficult to detect, difficult to extract information from, and is of relatively poor resolution.

Referring now to Fig. 5, electrical current i₂ drives LED 22. Current i₂ is greater than current i₁ of Fig. 4, causing the LED output of Fig. 5 to exceed the LED output of Fig. 4 as suggested by the more numerous wavelike arrows E. As a result the PD output 30 of Fig. 5 will exceed the PD output of Fig. 4. In principal, this makes the AC portion of signal of Fig. 5 easier to detect and easier to extract information from in comparison to the AC portion of the signal in Fig. 4. However, as seen by the dashed line 72 of the graph of Fig. 5, the potential benefit of the increased LED drive current is negated by the fact that the ADC 32 may be saturated by the PD output signal. This negation of the potential benefit of increased LED drive current is overcome by the bias current 62 provided to the PD by compensation current source 60. The bias current brings the AC component of the PD output signal back into the range of operation of the ADC. This is indicated by the solid line 74 of the graph of Fig. 5. As seen in Figs. 5 and 6, the magnitude of the bias current commanded by controller 50 depends on the PD output signal 30. The relationship between bias current 62 and PD output 30 is shown as linear in Fig. 6, but need not be.

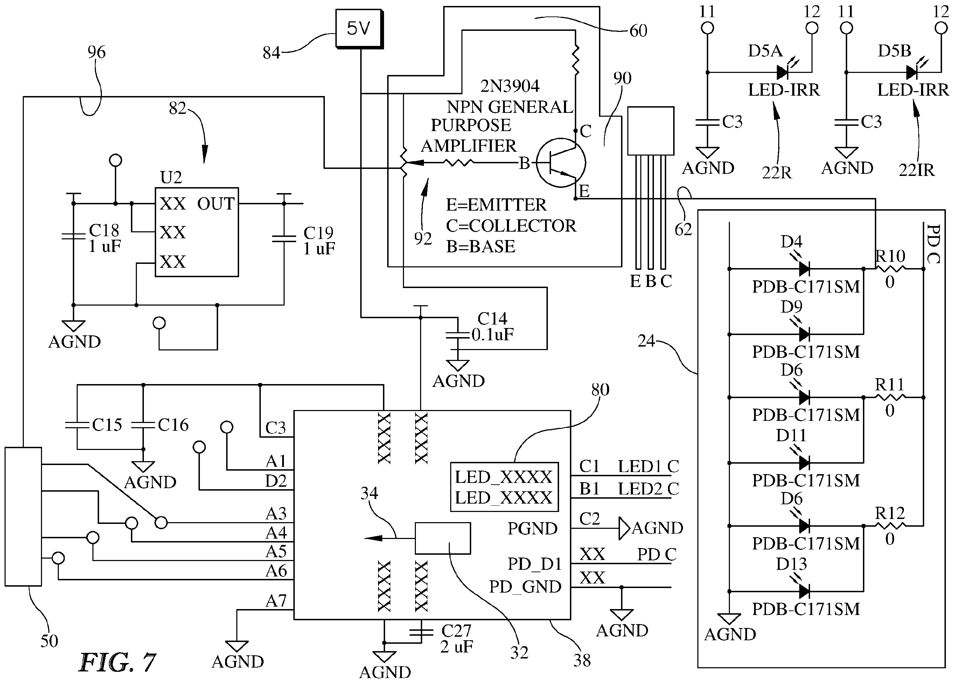

Fig. 7 is a diagram showing the components of Fig. 3 in more detail. The components include front end device 38, processor 50, red and infrared LED's 22R, 22IR, an LED driver 80 built into the front end device, an array of photodetectors 24 embodied as photodiodes, and ADC 32 built into the front end device. The diagram also illustrates a 1.8 volt power supply for the analogue section of the front end device and a 5 volt power supply for the digital components (LED drivers) of the front end device. One difference between Fig. 7 and Fig. 5 is that in Fig. 7 compensation current source 60 is an NPN transistor 90 controlled by an analogue potentiometer 92. A PNP transistor could be used instead, along with other modifications that would be evident to those skilled in the art in order to accommodate the PNP transistor. Either way the operation of the compensated PPG device is the same. Signal 96 from processor 50 adjusts the potentiometer in order to control electrical current from the emitter of the transistor. The current from the emitter of the transistor is the bias current 62 previously described.

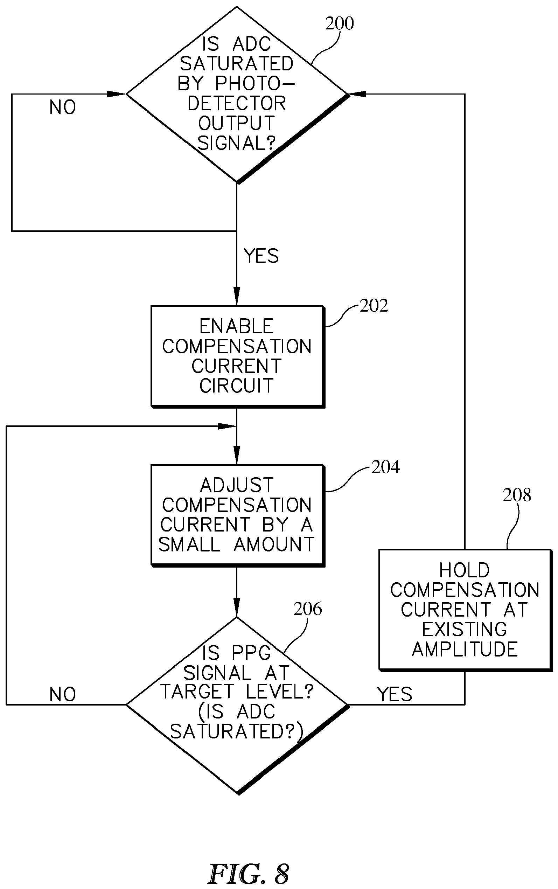

Fig. 8 is a block diagram illustrating a method of overcoming saturation of an analogue to digital converter of a PPG device. At block 200, processor 50, acting in accordance with machine readable instructions 52, assesses whether or not ADC 32 is saturated by photodetector output 30. If not the processor continues carrying out the step of block 200. If so, the processor advances to block 202.

At block 202, the processor enables the compensation current circuit 60. At block 204, the processor adjusts the compensation current by an increment and advances to block 206.

At block 206, the processor tests whether or not the photodetector output signal 30 is at a target level, i.e. at a level that causes the ADC to be unsaturated. The instructions 52 may be written to recognize gradations of saturation, in which case block 206 is interpreted as testing whether or not the photodetector output signal 30 is at a target level that renders the ADC sufficiently unsaturated, for example unsaturated enough to not be at the threshold or borderline of saturation.

If the test at block 206 reveals that the compensation current applied at block 204 has been effective at unsaturating the ADC, the method advances to block 208, which holds the compensation current at the amplitude resulting from the adjustment of block 204. The method then branches back to block 200. However, if the test at block 206 reveals that the compensation current applied at block 204 has not been effective (i.e. the ADC remains saturated), the method advances to block 204 and makes a further adjustment to the compensation current.

Although this disclosure refers to specific embodiments, it will be understood by those skilled in the art that various changes in form and detail may be made.

## Claims

1. A photoplesythmography (PPG) device comprising:
an array of light emitting diodes (LED's) arranged to illuminate a tissue sample;
an array of photodetectors (PD's) adapted to detect light returned from the tissue sample and to output a PD output signal which depends at least in part on a bias current;
a compensation current source; and
a controller adapted to operate the compensation current source so that the compensation current source outputs the bias current to the array of photodetectors.

2. The PPG device of claim 1, further comprising an analogue to digital converter (ADC) which processes the PD output signal, and wherein the controller controls the compensation current source so that a magnitude of the bias current is a function of the ADC output signal.

3. The PPG device of claim 2, wherein the controller controls the compensation current source such that the PD output signal is within a range of operation of the ADC.

4. The PPG device of any preceding claim, wherein the compensation current source comprises a digital to analogue converter (DAC).

5. The PPG device of any preceding claim, wherein the compensation current source comprises a digital potentiometer whose setting is controlled by the controller to regulate the bias current.

6. The PPG device of any preceding claim, wherein the compensation current source comprises a transistor, and the controller controls the transistor by way of a potentiometer in electrical communication with the transistor.

7. The PPG device of any preceding claim, wherein the controller is adapted to:
assess whether or not an analogue to digital converter (ADC) of the PPG device is saturated by the PD output signal;
if the assessment determines that the ADC is saturated, commanding the compensation current source to increment its output;
test whether or not the ADC has become unsaturated in response to the command to the compensation current source to increment its output; and
a) if the test reveals that the bias current resulting from the incremental adjustment has unsaturated the ADC, decline to command a further increment to the output of the compensation current source; and
b) if the test reveals that the bias current resulting from the incremental adjustment has not unsaturated the ADC, commanding a further incremental adjustment to the compensation current.

8. A method of overcoming saturation of an analogue to digital converter (ADC) of a photoplethsymography (PPG) device, the method comprising:
assessing whether or not the ADC is saturated by a photodetector output;
if the assessment determines that the ADC is saturated, adjusting a compensation current by an incremental amount;
testing whether or not the ADC has become unsaturated in response to the adjusting step; and
a) if the testing step reveals that the compensation current resulting from the incremental adjustment has unsaturated the ADC, holding the compensation current at the amplitude resulting from the incremental adjustment; and
b) if the testing step reveals that the compensation current resulting from the incremental adjustment has not unsaturated the ADC, making a further incremental adjustment to the compensation current.

9. The method of claim 8, further comprising illuminating a tissue sample with an array of light emitting diodes (LED's), detecting light returned from the tissue sample by an array of photodetectors (PD's), and outputting the photodetector output as a photodetector (PD) output signal.

10. The method of claim 9, further comprising outputting the compensation current from a compensation current source and using a controller to operate the compensation current source so that the compensation current source outputs the compensation current to the array of photodetectors.

11. The method of claim 10, further comprising controlling the compensation current source with the controller so that a magnitude of the compensation current is a function of an ADC output signal from the ADC.

12. The method of either claim 10 or claim 11, further comprising controlling the compensation current source with the controller such that the PD output signal is within a range of operation of the ADC.

13. The method of any one of claims 10 to 12, wherein the compensation current source comprises a digital to analogue converter (DAC).

14. The method of any one of claims 10 to 13, wherein the compensation current source comprises a digital potentiometer whose setting is controlled by the controller to regulate the compensation current.

15. The method of any one of claims 10 to 14, wherein the compensation current source comprises a transistor, and the controller controls the transistor by way of a potentiometer in electrical communication with the transistor.
